(19) **Europäisches Patentamt**
**European Patent Office**
**Office européen des brevets**

(11) **EP 1 815 002 B1**

(12) **EUROPÄISCHE PATENTSCHRIFT**

(45) Veröffentlichungstag und Bekanntmachung des Hinweises auf die Patenterteilung:
**08.04.2009 Patentblatt 2009/15**

(51) Int Cl.:
*C12P 7/16* (2006.01)    *C12N 9/04* (2006.01)
*C12P 41/00* (2006.01)

(21) Anmeldenummer: **05806536.8**

(22) Anmeldetag: **15.11.2005**

(86) Internationale Anmeldenummer:
**PCT/EP2005/012234**

(87) Internationale Veröffentlichungsnummer:
**WO 2006/053713 (26.05.2006 Gazette 2006/21)**

(54) **VERFAHREN ZUR HERSTELLUNG VON (S)-BUTAN-2-OL**

PROCESS FOR THE PREPARATION OF (S)-BUTAN-2-OL

PROCÉDÉ DE PRÉPARATION DE (S)-BUTAN-2-OL

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **17.11.2004 DE 102004055508**

(43) Veröffentlichungstag der Anmeldung:
**08.08.2007 Patentblatt 2007/32**

(73) Patentinhaber: **BASF SE**
**67056 Ludwigshafen (DE)**

(72) Erfinder:
• **STÜRMER, Rainer**
**67127 Rödersheim-Gronau (DE)**
• **HAUER, Bernhard**
**67136 Fussgönheim (DE)**
• **DREW, Dejana**
**68167 Mannheim (DE)**
• **BREUER, Michael**
**64285 Darmstadt (DE)**
• **SCHRÖDER, Hartwig**
**69226 Nussloch (DE)**

(56) Entgegenhaltungen:
**US-A- 5 763 236**

• **VELONIA K ET AL: "Stereospecificity of Hydrogen Transfer by the NAD<+>-linked Alcohol Dehydrogenase from the Antarctic Psychrophile Moraxella sp. TAE123" BIOORGANIC & MEDICINAL CHEMISTRY LETTERS, OXFORD, GB, Bd. 9, Nr. 1, Januar 1999 (1999-01), Seiten 65-68, XP004154779 ISSN: 0960-894X**

• **MATSUDA T ET AL: "Asymmetric reduction of simple aliphatic ketones with dried cells of Geotrichum candidum" TETRAHEDRON: ASYMMETRY, ELSEVIER SCIENCE PUBLISHERS, AMSTERDAM, NL, Bd. 13, Nr. 9, 24. Juni 2002 (2002-06-24), Seiten 971-974, XP004365921 ISSN: 0957-4166 in der Anmeldung erwähnt**

• **CURT W BRADSHAW ET AL: "A Pseudomonas sp. alcohol dehydrogenase with broad substrate specificity and unusual stereospecificity for organic synthesis" JOURNAL OF ORGANIC CHEMISTRY, AMERICAN CHEMICAL SOCIETY. EASTON, US, Bd. 57, Nr. 5, 28. Februar 1992 (1992-02-28), Seiten 1526-1532, XP002024201 ISSN: 0022-3263 in der Anmeldung erwähnt**

• **KEINAN ET AL.: "Thermostable enzymes in organic synthesis. 2.1 Asymmetric reduction of ketones with alcohol dehydrogenase from Thermoanaerobium brockii" J. AM. CHEM. SOC., Bd. 108, 1986, Seiten 162-169, XP002371515 in der Anmeldung erwähnt**

• **DATABASE Geneseq [Online] 14. Februar 2002 (2002-02-14), "E. coli cellular proliferation protein #150." XP002371518 gefunden im EBI accession no. GSP:AAU34569 Database accession no. AAU34569 & WO 01/70955 A (ELITRA PHARMACEUTICALS, INC; HASELBECK, ROBERT; OHLSEN, KARI, L; ZYSKI) 27. September 2001 (2001-09-27)**

- DATABASE UniProt [Online] 1. November 1997 (1997-11-01), "Alcohol dehydrogenase, propanol-preferring (EC 1.1.1.1)." XP002371519 gefunden im EBI accession no. UNIPROT:ADHP_ECOLI Database accession no. P39451 in der Anmeldung erwähnt & BLATTNER F R ET AL: "THE COMPLETE GENOME SEQUENCE OF ESCHERICHIA COLI K-12" SCIENCE, AMERICAN ASSOCIATION FOR THE ADVANCEMENT OF SCIENCE,, US, Bd. 277, 5. September 1997 (1997-09-05), Seiten 1453-1462, XP002069950 ISSN: 0036-8075

**Beschreibung**

Die vorliegende Erfindung betrifft ein Verfahren zur Herstellung von (S)-Butan-2-ol durch Reduktion von Butan-2-on in Gegenwart einer Alkohol-Dehydrogenase.

Stand der Technik

Die Reduktion von Butan-2-on zu Butan-2-ol mit verschiedenen Biokatalysatoren ist beschrieben [J.Org.Chem. (1992) 57:1526, Chemistry Letters (1987) 2089, Arch.Biochem Biophys. (1992) 292:539].

In diesen Arbeiten sind allerdings keinerlei Enantioselektivitäten angegeben. Des wéiteren ist die Herstellung von *R*-Butan-2-ol veröffentlicht [J.Am.Chem.Soc. (1986) 108:162]. Hier sind aber nur Enantioselektivitäten von 48% ee erhalten worden.

Die biokatalytische Synthese von S-Butan-2-ol ist von Nakamura et al. gezeigt worden [Tetrahedron: Asymmetry (2003) 14:2659, Tetrahedron: Asymmetry (2002) 13:971] Enantioselektivitäten von 94% ee wurden erzielt. In diesen Arbeiten wird mit getrockneten Zellen von *Geotrichum candidum* gearbeitet. Das Enzym, das die obenstehende Reaktion katalysiert, ist allerdings nicht näher beschrieben.

Das Dokument VELONIA, K. ET AL.: "Stereospecificity of Hydrogen Transfer by the NAD+-linked Alcohol Dehydrogenase from the Antarctic Psychrophile Moraxella sp. TAE123" (BIOORGANIC & MEDICINAL CHEMISTRY LETTERS, Bd. 9, Nr. 1, Januar 1999, Seiten 65-68) offenbart eine bakterielle Alkohol-Dehydrogenase aus *Moraxella* sp. TAE123 und deren Verwendung zur Herstellung von (S)-Butan-2-ol durch Reduktion von Butan-2-on.

Aufgabenstellung

Es bestand die Aufgabe, ein Verfahren zur Herstellung von (S)-Butan-2-ol durch enantioselektive Reduktion von Butan-2-on bereitzustellen, das in einer hohen chemischen Ausbeute (S)-Butan-2-ol möglichst enantiomerenrein liefert.

Beschreibung der Erfindung

Die vorliegende Erfindung betrifft ein Verfahren zur Herstellung von (S)-Butan-2-ol durch Reduktion von Butan-2-on in Gegenwart einer Alkohol-Dehydrogenase

(i) mit der Polypeptidsequenz SEQ ID NO:2, oder
(ii) mit einer Polypeptidsequenz, die mindestens 80% Sequenzidentität mit SEQ ID NO:2 aufweist.

Für das erfindungsgemässe Verfahren geeignete Alkohol-Dehydrogenasen (im folgenden mit ADH abgekürzt) sind solche ADH, die in der Lage sind, 2-Propanol in einer NAD$^+$ abhängigen Reaktion zu 2-Propanon zu oxidieren.

Weiterhin besitzen die für das erfindungsgemäße Verfahren geeigneten ADH eine Polypeptidsequenz gemäss SEQ ID NO:2 oder eine Polypeptidsequenz, die mindestens 80%, bevorzugt mindestens 90%, besonders bevorzugt mindestens 95% und insbesondere mindestens 97%, 98% oder 99% Sequenzidentität mit SEQ ID NO:2 aufweist.

Ein Polypeptid mit der SEQ ID NO:2 ist die aus Escherichia coli erhältliche Propanol-Dehydrogenase [Swissprot: locus ADHP_ECOLI, accession P39451; Genbank ID 48994873 Region: complement (1550852 bis 1551892; [Science (1997) 277:1453]. Die Isolierung dieses Enzyms ist im experimentellen Teil beschrieben.

Weitere geeignete ADH sind solche, deren Polypeptidsequenz mit SEQ ID NO:2 eine der oben genannten Sequenzidentitäten aufweist.

Die Ermittlung der Sequenzidentität soll für die hier beschriebenen Zwecke durch das Computerprogramm "GAP" der Genetics Computer Group (GCG) der University of Wisconsin erfolgen, wobei die Version 10.3 unter Verwendung der von GCG empfohlenen Standardparameter zum Einsatz kommen soll.

Solche ADH können ausgehend von SEQ ID NO:2 durch dem Fachmann bekannte gezielte oder randomisierte Mutageneseverfahren erhalten werden. Alternativ kann jedoch auch in Mikroorganismen , bevorzugt in solchen der Gattungen *Alishewanella, Alterococcus, Aquamonas, Aranicola, Arsenophonus, Azotivirga, Brenneria, Buchnera (aphid P-endosymbionts), Budvicia, Buttiauxella, Candidatus Phlomobacter, Cedecea, Citrobacter, Dickeya, Edwardsiella, Enterobacter, Erwinia, Escherichia, Ewingella, Grimontella, Hafnia, Klebsiella, Kluyvera, Leclercia, Leminorella, Moellerella, Morganella, Obesumbacterium, Pantoea, Pectobacterium, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworlhia, Xenorhabdus, Yersinia* oder *Yokenel*la, nach ADH gesucht werden, die die entsprechende Oxidation von 2-Propanol zu 2-Propanon katalysieren und deren Aminosäuresequenz die geforderte Sequenzidentität zu SEQ ID NO:2 bereits aufweist oder durch Mutageneseverfahren erhalten wird.

Die ADH kann in gereinigter oder teilweise gereinigter Form oder auch in Form des Mikroorganismus selbst verwendet werden. Verfahren zur Gewinnung und Aufreinigung von Dehydrogenasen aus Mikroorganismen sind dem Fachmann hinreichend bekannt, z.B. aus K. Nakamura & T. Matsuda, "Reduction of Ketones" in K. Drauz und H. Waldmann, Enzyme Catalysis in Organic Synthesis 2002, Vol.III, 991-1032, Wiley-VCH, Weinheim. Rekombinante Verfahren zur Erzeugung von Dehydrogenasen sind ebenfalls bekannt, beispielsweise aus W. Hummel, K. Abokitse, K. Drauz, C. Rollmann und H. Gröger, Adv. Synth. Catal. 2003, 345, Nr. 1 + 2, S. 153-159.

Bevorzugt erfolgt die die enantioselektive Reduktion mit der ADH in Gegenwart eines geeigneten Cofaktors (auch als Cosubstrat bezeichnet). Als Cofaktoren für die Reduktion des Ketons dient üblicherweise NADH und/oder NADPH. Daneben können ADH als zelluläre Systeme eingesetzt werden, die inherent Cofaktor enthalten, oder alternative Redoxmediatoren zugesetzt werden (A. Schmidt, F. Hollmann und B. Bühler "Oxidation of Alcohols" in K. Drauz und H. Waldmann, Enzyme Catalysis in Organic Synthesis 2002, Vol.III, 991-1032, Wiley-VCH, Weinheim).

Bevorzugt erfolgt die enantioselektive Reduktion mit der ADH außerdem in Gegenwart eines geeigneten Reduktionsmittels, welches den im Verlauf der Reduktion oxidierten Cofaktor regeneriert. Beispiele für geeignete Reduktionsmittels sind Zucker, insbesondere Hexosen, wie Glucose, Mannose, Fructose, und/oder oxidierbare Alkohole, insbesondere Ethanol, Propanol oder Isopropanol, sowie Formiat, Phosphit oder molekularer Wasserstoff. Zur Oxidation des Reduktionsmittels und damit verbunden zur Regeneration des Coenzyms kann eine zweite Dehydrogenase, wie z.B. Glucosedehydrogenase bei Verwendung von Glucose als Reduktionsmittel oder Formiat-Dehydrogenase bei der Verwendung von Formiat als Reduktionsmittel, zugesetzt werden. Diese kann als freies oder immobilisiertes Enzym oder in Form von freien oder immobilisierten Zellen eingesetzt werden. Ihre Herstellung kann sowohl separat als auch durch Coexpression in einem (rekombinanten) Dehydrogenase-Stamm erfolgen.

Eine bevorzugte Ausführungsform des beanspruchten Verfahrens ist die Regenerierung der Cofaktoren durch ein enzymatisches System, bei dem eine zweite Dehydrogenase, besonders bevorzugt eine Glucosedehydrogenase, verwendet wird.

Verwendung könnte die ADH auch zur Herstellung von (R)-Butan-2-olfinden. Bei diesem Verfahren wird racemisches Butan-2-ol mit ADH umgesetzt, wobei selektiv das (S)-Butan-2-ol zu Butan-2-on oxidiert wird, während das (R)-Butan-2-ol unverändert bleibt. Anschliessend lässt sich das erhaltene Gemisch aus Butan-2-on und (R)-Butan-2-ol durch übliche Verfahren, beispielsweise durch Destillation, auftrennen und somit das (R)-Butan-2-ol in reiner Form isolieren. Hier werden bevorzugt NAD$^+$ bzw. NADP$^+$ als Cofaktoren verwendet, die mit entsprechenden Cosubstraten (Oxidationsmitteln) wieder regeneriert werden können. Als Cosubstrat kann hier bevorzugt Aceton eingesetzt werden, das mit der bereits vorhandenen ADH und/oder einer zusätzlich verwendeten Dehydrogenase den Cofaktor regeneriert, wobei es zum Isopropanol reduziert wird.

"Enantioselektivität" im Rahmen der vorliegenden Erfindung bedeutet, dass der Enantiomerenüberschuss ee (in %) des S-Enantiomers, der sich in bekannter Weise berechnet nach:

$$\text{ee (\%)} = (\text{S-Enantiomer} - \text{R-Enantiomer}) / (\text{S-Enantiomer} + \text{R-Enantiomer}) \times 100$$

wenigstens 80 %, vorzugsweise wenigstens 90 %, insbesondere wenigstens 95 % und speziell wenigstens 97 % beträgt.

Die erfindungsgemäß verwendeten ADH können frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS-10019377 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" in K. Drauz und H. Waldmann, Enzyme Catalysis in Organic Synthesis 2002, Vol.III, 991-1032, Wiley-VCH, Weinheim beschrieben.

Die Umsetzung (Reduktion des Butan-2-on zu Butan-2-ol) kann in wässrigen oder nichtwässrigen Reaktionsmedien oder in 2-Phasensystemen oder (Mikro-)Emulsionen erfolgen. Bei den wässrigen Reaktionsmedien handelt es sich vorzugsweise um gepufferte Lösungen, die in der Regel einen pH-Wert von 4 bis 8, vorzugsweise von 5 bis 8, aufweisen. Das wässrige Lösungsmittel kann neben Wasser außerdem wenigstens einen Alkohol, z.B. Ethanol oder Isopropanol oder Dimethylsulfoxid enthalten.

Unter nicht-wässrigen Reaktionsmedien werden Reaktionsmedien verstanden, die weniger als 1 Gew.-%, vorzugsweise weniger als 0,5 Gew.-% Wasser, bezogen auf das Gesamtgewicht des Reaktionsmediums, enthalten. Vorzugsweise wird die Umsetzung in einem organischen Lösungsmittel durchgeführt.

Geeignete Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Ether, insbesondere Tetrahydrofuran, verwendet.

Bevorzugt wird die Reduktion mit der ADH in einem wässrig-organischen, insbesondere wässrigen Reaktionsmedium durchgeführt.

Das Butan-2-on wird vorzugsweise in einer Konzentration von 0,1 g/l bis 500 g/l, besonders bevorzugt von 1 g/l bis 50 g/l in die enzymatische Reduktion eingesetzt und kann kontinuierlich oder diskontinuierlich nachgeführt werden.

Die enzymatische Reduktion erfolgt in der Regel bei einer Reaktionstemperatur unterhalb der Desaktivierungstemperatur der eingesetzten Dehydrogenase und oberhalb von -10°C. Besonders bevorzugt liegt sie im Bereich von 0 bis 100°C, insbesondere von 15 bis 60°C und speziell von 20 bis 40°C, z.B. bei etwa 30°C.

Zur Durchführung kann man beispielsweise das Butan-2-on mit der ADH, dem Lösungsmittel und gegebenenfalls den Coenzymen, gegebenenfalls einer zweiten Dehydrogenase zur Regenerierung des Coenzyms und/oder weiteren Reduktionsmitteln vorlegen und das Gemisch durchmischen, z. B. durch Rühren oder Schütteln. Es ist aber auch möglih, die Dehydrogenase(n) in einem Reaktor, beispielsweise in einer Säule, zu immobilisieren, und durch den Reaktor eine das Butan-2-on und gegebenenfalls Coenzyme und/oder Cosubstrate enthaltende Mischung zu leiten. Hierzu kann man die Mischung im Kreislauf durch den Reaktor leiten bis der gewünschte Umsatz erreicht ist. Dabei wird die Ketogruppe des Butan-2-on einer OH-Gruppe reduziert, wobei im Wesentlichen das (S)-Enantiomere des Alkohols entsteht. In der Regel wird man die Reduktion bis zu einem Umsatz von wenigstens 70 %, besonders bevorzugt von wenigstens 85 % und insbesondere von wenigstens 95%, bezogen auf das in der Mischung enthaltene Butan-2-on führen. Das Fortschreiten der Reaktion, d. h. die sequentielle Reduktion des Ketons, kann dabei durch übliche Methoden wie Gaschromatographie oder Hochdruckflüssigkeitschromatographie verfolgt werden.

Erfindungsgemäß mit umfasst ist die Verwendung von "funktionalen Äquivalenten" der konkret offenbarten Enzyme mit Butan-2-ol-Dehydrogenase-Aktivität.

"Funktionale Äquivalente" oder Analoga der konkret offenbarten Enzyme sind davon verschiedene Polypeptide, welche weiterhin die gewünschte biologische Aktivität, wie z.B. Substratspezifität, besitzen. So versteht man beispielsweise unter "funktionalen Äquivalenten" Enzyme, die von Butan-2-on zum entsprechenden S-Alkohol reduzieren und die mindestens 20 %, bevorzugt 50 %, besonders bevorzugt 75 %, ganz besonders bevorzugt 90 % der Aktivität eines Enzyms, umfassend eine der unter SEQ ID NO:2 aufgeführten Aminosäuresequenzen, aufweist. Funktionale Äquivalente sind außerdem vorzugsweise zwischen pH 4 bis 10 stabil und besitzen vorteilhaft ein pH-Optimum zwischen pH 5 und 8 sowie ein Temperaturoptimum im Bereich von 20°C bis 80°C.

Unter "funktionalen Äquivalenten" versteht man insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |

(fortgesetzt)

| Ursprünglicher Rest | Beispiele der Substitution |
|---|---|
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate".

"Präkursoren" sind dabei natürliche oder synthetische Vorstufen der Polypeptide mit oder ohne der gewünschten biologischen Aktiviät.

"Funktionale Derivate" der Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

Im Falle einer möglichen Proteinglykosylierung umfassen "funktionale Äquivalente" Proteine des oben bezeichneten Typs in deglykosylierter bzw. glykosylierter Form sowie durch Veränderung des Glykosylierungsmusters erhältliche abgewandelte Formen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Homologe der Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Offenbart sind Nukleinsäuresequenzen (einzel- und doppelsträngige DNA- und RNA-Sequenzen, wie z.B. cDNA und mRNA), die für ein Enzym mit Dehydrogenase-Aktivität kodieren. Bevorzugt sind Nukleinsäuresequenzen, welche z.B. für Aminosäuresequenzen gemäß SEQ ID NO:2 oder charakteristische Teilsequenzen davon kodieren, oder Nukleinsäuresequenzen gemäß SEQ ID NO:1 oder charakteristische Teilsequenzen davon umfassen.

Alle hierin erwähnten Nukleinsäuresequenzen sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Offenbart sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalenten, welche z.B. unter Verwendung künstlicher Nukleotidanaloga zugänglich sind.

Offenbart sind sowohl isolierte Nukleinsäuremoleküle, welche für Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von kodierenden Nukleinsäuren verwendet werden können.

Die Nukleinsäuremoleküle können zudem untranslatierte Sequenzen vom 3'- und/oder 5'-Ende des kodierenden Genbereichs enthalten

Offenbart sind weiterhin die zu den konkret beschriebenen Nukleotidsequenzen komplementären Nukleinsäuremoleküle oder einen Abschnitt davon.

Die Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein Nukleinsäuremolekül kann mittels molekularbiologischer Standard-Techniken und der bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die Oligonukleotide können ferner durch Standard-Syntheseverfahren, z.B. mit einem automatischen DNA-Synthesegerät, hergestellt werden.

Die Nukleinsäuresequenzen lassen sich prinzipiell aus allen Organismen identifizieren und isolieren. Vorteilhaft lassen sich die Nukleinsäuresequenzen oder die Homologen davon, aus Pilzen, Hefen, Archeen oder Bakterien isolieren. Als Bakterien seien gram-negative und gram-positive Bakterien genannt. Bevorzugt werden die Nukleinsäuren aus gram-negativen Bakterien vorteilhaft aus α-Proteobakterien, β-Proteobakterien oder γ-Proteobakterien, besonders bevorzugt aus Bakterien der Ordnungen der *Acidithiobacillales, Alteromonadales, Chromatiales, Enterobacteriales, Legionellales, Methylococcales, Oceanospirillales, Pasteurellales; Pseudomonadales, Thiotrichales, Vibrionales, Xanthomonadales.* Ganz besonders bevorzugt aus Bakterien der Familie der *Enterobacteriaceae*. Insbesondere bevorzugt aus der Gattung *Escherichia*. Insbesondere bevorzugt aus Arten *Escherichia albertii, Escherichia blattae, Escherichia coli, Escherichia fergusonii, Escherichia hermannii, Escherichia senegalensis* und *Escherichia vulneris*.

Besonders bevorzugt verwendet man Dehydrogenasen aus *Escherichia coli.*

Nukleinsäuresequenzen lassen sich beispielsweise mit üblichen Hybridisierungsverfahren oder der PCR-Technik aus anderen Organismen, z.B. über genomische oder cDNA-Banken, isolieren. Diese DNA-Sequenzen hybridisieren unter Standardbedingungen mit den Sequenzen. Zur Hybridisierung werden vorteilhaft kurze Oligonukleotide der konservierten Bereiche beispielsweise aus dem aktiven Zentrum, die über Vergleiche mit einer Dehydrogenase in dem Fachmann bekannter Weise ermittelt werden können, verwendet. Es können aber auch längere Fragmente der Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10˚C niedriger als die von DNA:RNA-Hybriden gleicher Länge.

Unter Standardbedingungen sind beispielsweise je nach Nukleinsäure Temperaturen zwischen 42 und 58˚C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42˚C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20˚C bis 45˚C, bevorzugt zwischen etwa 30˚C bis 45˚C. Für DNA:RNA-Hybride liegen die Hybridisierungsbedingungen vorteilhaft bei 0,1 x SSC und Temperaturen zwischen etwa 30˚C bis 55˚C, bevorzugt zwischen etwa 45˚C bis 55˚C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Offenbart sind auch Derivate der konkret offenbarten oder ableitbaren Nukleinsäuresequenzen.

So können weitere Nukleinsäuresequenzen von SEQ ID NO:1 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Offenbart sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Offenbart sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Offenbart sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Außerdem sind unter Derivaten beispielsweise Fusionen mit Promotoren zu verstehen. Die Promotoren, die den angegebenen Nukleotidsequenzen vorgeschalten sind, können durch ein oder mehrere Nukleotidaustausche, Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch Varianten zu verstehen, deren Nukleotidsequenz im Bereich von -1 bis -1000 Basen stromaufwärts vor dem Startkodon oder 0 bis 1000 Basen stromabwärts nach dem Stopkodon so verändert wurden, dass die Genexpression und/oder die Proteinexpression verändert, bevorzugt erhöht wird.

Offenbart sind auch Nukleinsäuresequenzen, welchen mit oben genannten kodierenden Sequenzen unter "stringenten Bedingungen" hybridisieren. Diese Polynukleotide lassen sich bei der Durchmusterung von genomischen oder cDNA-Banken auffinden und gegebenenfalls daraus mit geeigneten Primern mittels PCR vermehren und anschließend beispielsweise mit geeigneten Sonden isolieren. Darüber hinaus können Polynukleotide auch auf chemischem Wege synthetisiert werden. Unter dieser Eigenschaft versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 ˚C, vorzugsweise 60 - 65 ˚C warmen Waschlösung, beispielsweise 0,1x SSC-Puffer mit 0.1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z:B. in Ausubel et al., Current Protocols in Molecular Biology, John Wiley & Sons, N.Y. (1989), 6.3.1-6.3.6. beschrieben.

Ausgestaltungen der Konstrukte

Offenbart sind außerdem Expressionskonstrukte, enthaltend unter der genetischen Kontrolle regulativer Nukleinsäuresequenzen eine für ein Polypeptid kodierende Nukleinsäuresequenz; sowie Vektoren, umfassend wenigstens eines dieser Expressionskonstrukte.

Vorzugsweise umfassen solche Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Unter einem Nukleinsäurekonstrukt sind insbesondere solche zu verstehen, bei weichen das Gen für eine Dehydrogenase mit einem oder mehreren Regulationssignalen zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für das erfindungsgemäße Verfahren sind beispielsweise in Promotoren wie cos-, tac-, trp-, tet-, trp-tet-, Ipp-, lac-, Ipp-lac-, lacl$^{q-}$, T7-, T5-, T3-, gal-, trc-, ara-, rhaP (rhaP$_{BAD}$)SP6-, lambda-P$_R$- oder im lambda-P$_L$-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha, AC, P-60, CYC1, GAPDH, TEF, rp28, ADH enthalten. In diesem Zusammenhang sind auch die Promotoren der Pyruvatdecarboxylase und der Methanoloxidase, beispielsweiseaus Hansenula vorteilhaft. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar. Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III$^{113}$-B1, Igt11 oder pBdC1, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, pGHlac$^+$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

Vorteilhafterweise enthält das Nukleinsäurekonstrukt zur Expression der weiteren enthaltenen Gene zusätzlich noch 3'- und/oder 5'-terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das Nukleinsäurekonstrukt oder die Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der Nukleinsäure bestehen.

Für eine optimale Expression heterologer Gene in Organisnien ist es vorteilhaft die Nukleinsäuresequenzen entsprechend des im Organismus verwendeten spezifischen "codon usage" zu verändern. Der "codon usage" läßt sich anhand von Computerauswertungen anderer, bekannter Gene des betreffenden Organismus leicht ermitteln.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

Brauchbare Wirtsorganismen

Mit Hilfe der Vektoren oder Konstrukte sind rekombinante Mikroorganismen herstellbar, welche beispielsweise mit wenigstens einem Vektor transformiert sind und zur Produktion der Polypeptide eingesetzt werden können. Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Es sind auch homolog rekombinierte Mikroorganismen herstellbar. Dazu wird ein Vektor hergestellt, der zumindest einen Abschnitt eines Gens oder einer kodierenden Sequenz enthält, worin gegebenenfalls wenigstens eine Aminosäure-Deletion, -Addition oder -Substitution eingebracht worden ist, um die Sequenz zu verändern, z.B. funktionell zu disrumpieren ("Knockout"-Vektor). Die eingebrachte Sequenz kann z.B. auch ein Homologes aus einem verwandten Mikroorganismus sein oder aus einer Säugetier-, Hefe- oder Insektenquelle abgeleitet sein. Der zur homologen Rekombination verwendete Vektor kann alternativ derart ausgestaltet sein, daß das endogene Gen bei homologer Rekombination mutiert oder anderweitig verändert ist, jedoch noch das funktionelle Protein kodiert (z.B. kann der stromaufwärts gelegene regulatorische Bereich derart verändert sein, dass dadurch die Expression des endogenen Proteins verändert wird). Der veränderte Abschnitt des Gens ist im homologen Rekombinationsvektor. Die Konstruktion geeigneter Vektoren zur homologen Rekombination ist z.B. beschrieben in Thomas, K.R. und Capecchi, M.R. (1987) Cell 51:503.

Als rekombinante Wirtsorganismen für die Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden.

Der Wirtsorganismus oder die Wirtsorganismen enthalten dabei vorzugsweise mindestens eine der beschriebenen Nukleinsäuresequenzen, Nukleinsäurekonstrukte oder Vektoren, die für ein Enzym mit Dehydrogenaseaktivität kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0°C und 100°C, bevorzugt zwischen 10°C bis 60°C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden. Das Keton kann direkt zur Anzucht gegeben werden oder vorteilhaft nach Anzucht. Die Enzyme können nach dem in den Beispielen beschriebenen Verfahren aus den Organismen isoliert werden oder als Rohextrakt für die Reaktion verwendet werden.

Rekombinante Herstellung der erfindungsgemäß verwendeten Polypeptide

Offenbart sind weiterhin Verfahren zur rekombinanten Herstellung der erfindungsgemäß verwendeten Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Der rekombinante Mikroorganismus kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

Weitere Ausgestaltungen zur Durchführung des erfindungsgemäßen enzymatischen Reduktionsverfahrens

Die Dehydrogenasen können im erfindungsgemäßen Verfahren als freies oder immobilisiertes Enzym verwendet werden.

Das erfindungsgemäße Verfahren wird vorteilhaft bei einer Temperatur zwischen 0°C bis 95°C, bevorzugt zwischen 10°C bis 85°C, besonders bevorzugt zwischen 15°C bis 75°C durchgeführt.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten.

Unter enantiomerenreinen bzw. chiralen Produkten, wie Butan-2-ol, sind im erfindungsgemäßen Verfahren Enantiomere zu verstehen, die eine Enantiomerenanreicherung zeigen. Bevorzugt werden im Verfahren Enantiomerenreinheiten von mindestens 70 %ee, bevorzugt von min. 80 %ee, besonders bevorzugt von min. 90 %ee, ganz besonders bevorzugt min. 98 %ee erreicht.

Für das erfindungsgemäße Verfahren können wachsende Zellen verwendet werden, die die Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die nachfolgenden Beispiele sollen die Erfindung veranschaulichen. Hierbei wird auf beiliegende Abbildung Bezug genommen, dabei zeigt:

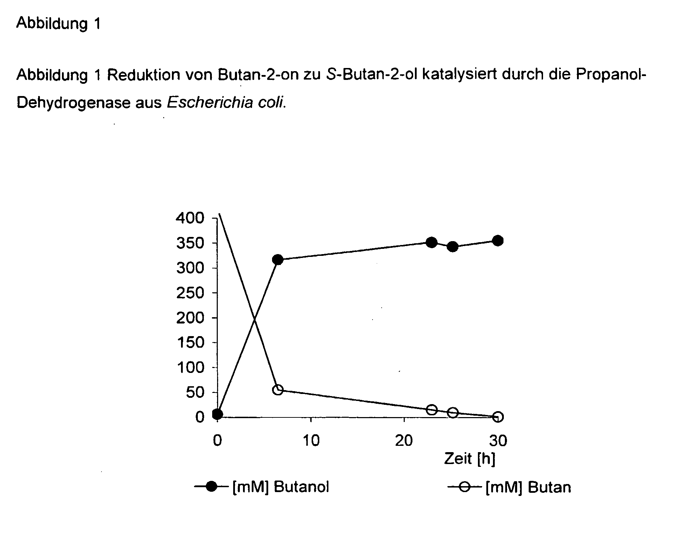

Abbildung 1 zeigt einen typische Reaktionsverlauf einer Synthese von (S)-Butan-2-ol durch Reduktion mit einer Dehydrogenase aus *Escherichia coli*.

Experimenteller Teil

Beispiel 1: Klonierung der Propanol-Dehydrogenase aus *Escherichia coli*.

Die Sequenz des Propanol-Dehydrogenasegens aus *Escherichia coli* ist in Datenbanken hinterlegt (Genbank ID 48994873 Region: complement (1550852 bis 1551892)). Von der Nukleinsäuresequenz des Propanol-Dehydrogenase-Gens wurden Oligonukleotide abgeleitet, mit denen nach bekannten Verfahren das Gen aus genomischer DNA von *Escherichia coli* amplifiziert wurde. Die erhaltene Sequenz entspricht der publizierten Sequenz. Die DNA-Sequenz der Oligonukleotide ist in Tabelle 1 dargestellt.

Tabelle 1 Oligonukleotide zur Herstellung des synthetischen Propanol-Dehydrogenase-Gens aus *Escherichia coli*

| #1 | GGGAATTCATATGCAGAACATCATCCGAAAAG |
|----|----------------------------------|
| #2 | CCCAAGCTTAGTGACGGAAATCAATC |

PCR-Bedingungen:

| 2 $\mu$L | 10*Pfu-Ultra Puffer (Stratagene®) |
|---|---|
| 100 ng | Primer #1 ( vgl. Tabelle 1) |
| 100 ng | Primer #2 ( vgl. Tabelle 1) |
| 1 $\mu$L | dNTP (je 10 mM) |
| ca. 30 ng | chromosomale DNA aus *Escherichia coli* |
| 1 U | Pfu-Ultra DNA Polymerase |
| ad 20 $\mu$L | H$_2$O |

Temperaturprogramm:

5 min, 95˚C,
45 sec, 40˚C,
1,5 min, 72˚C,  } (30 Zyklen)
45 sec, 95˚C,

(fortgesetzt)

10 min, 72˚C,
∞, 10˚C

Das PCR-Produkt (ca. 1050bp) wurde mit den Restriktionsendonukleasen *Nde*I und *Hind*III verdaut und in entsprechend v geschnittene pDHE19.2-Vektor kloniert (die Klonierung erfolgte wie in DE 19848129 beschrieben). Die Ligationsansätze wurden in E.coli XL1 Blue (Stratagene®) transformiert.

Das erhaltene Plasmid *p*DHE-PDH-L wurde in den Stamm *E.coli* TG10 pAgro4 pHSG575 transformiert (TG10: ein RhaA⁻-Derivat von E.coli TG1 (Stratagene®); pAgro4: Takeshita, S; Sato, M; Toba, M; Masahashi, W; Hashimoto-Gotoh, T (1987) Gene 61, 63-74; pHSG575: T. Tomoyasu et al (2001), Mol. Microbiol. 40(2), 397-413). Der erhaltene rekombiante E. *coli* Klon wurde mit E. coli LU12418 bezeichnet.

Beispiel 2: Bereitstellung rekombinanter Propanol-Dehydrogenase

E.coli LU12418 wurden in 20mL LB-Amp/Spec/Cm (100µg/l Ampicillin; 100µg/l Spectinomycin; 20µg/l Chloramphenicol), 0,1mM IPTG, 0,5g/L Rhamnose in 100mL Erlenmeyerkolben (Schikanen) 18 h bei 37˚C angezogen, bei 5000*g/10min zentrifugiert, einmal mit 10mM TRIS*HCl, pH7,0 gewaschen und in 2 mL des gleichen Puffers resuspendiert.

Zellfreier Proteinrohextrakt wurde hergestellt indem Zellpaste von E.coli LU12418 0,7ml Glaskugeln (d=0,5mm) in einer Schwingmühle (3x 5min mit Zwischenkühlung auf Eis) aufgeschlossen wurde.

Beispiel 3: Aktivitätsbestimmung der rekombinanten Dehydrogenase aus E.coli LU12418

Je 6 Transformanten wurden in 20mL LBAmp/Spec/Cm (100µg/l Amp; 100mg/ISpec; 20µg/ICm) 0,1mM IPTG 0,5g/L Rhamnose in 100mL Erlenmeyerkolben (Schikanen) 18 h bei 37˚C angezogen, bei 5000*g/10min zentrifugiert, einmal mit 10mM Tris/HCl pH7,0 gewaschen und in 2 mL des gleichen Puffers resuspendiert.

Zellfreier Rohextrakt von *E. coli* LU12418 wurde durch Zellaufschluß mit 0,7ml Glaskugeln (d=0,5mm) in einer Schwingmühle (3x 5min mit Zwischenkühlung auf Eis) gewonnen.

Im Photometer kann bei 340 nm der Verbrauch reduzierter Kosubstrate während der Reduktion von Ketonen verfolgt werden. In 1 mL 50 mM KP$_i$, 1 mM M$_9$Cl$_2$, pH 6.5 wurden bei 30˚ C 10 µL verdünnter zellfreier Rohextrakt ($\cong$ 10 µg Protein), 10 µmol n-Propanal und 250 nmol NADH oder NADPH inkubiert. 1 Unit (1 U) entspricht der Enzymmenge, die in 1 min 1µmol n-Propanal reduziert.

Beispiel 4: Analytik von Butan-2-ol

Die Konzentration von Butan-2-on und Butan-2-ol lassen sich mittels GC bestimmen. Je nach Wahl der stationären und mobilen Phase lassen sich neben der Konzentration auch ee-Wert bestimmen.

a) achirale Analytik

Die Quantifizierung der Umsetzung wurde mit folgendem System durchgeführt:

| | |
|---|---|
| stationäre Phase: | Chromoltih SpeedROD RP18, 50*4, 6µm, Merck (Darmstadt) temperiert auf 45˚C |
| mobilePhase: | Laufmittel A: 10mM KH$_2$PO$_4$, pH 2.5 |
| | Laufmittel B: Acetonitril |
| | Gradient: 0-0.5 min, 35%B; 0.5-1.0 min 35 auf 80%B; 1.0-1.2 min 80%B; 1.2-1.3 min 80% - 35%B; 1.3-2.0 min 35%B; |
| Flussrate: | 1.5 ml/min |
| Detektion: | UV-Detektion bei 230 und 260nm |
| Retentionszeiten: | Butan-2-on: ca. 1.6 min |
| | Butan-2-ol: ca. 1.3 min |

Mit authentischem Material wird eine Eichreihe erstellt, anhand derer die Konzentration unbekannter Proben bestimmt werden kann.

b) chirale Analytik

| | |
|---|---|
| stationäre Phase: | Chiracel OD-H, 250*4, 6$\mu$m, Daicel, temperiert auf 40°C |
| mobilePhase: | Laufmittel A: n-Hexan |
| | Laufmittel B: *iso*-Propanol |
| | isokratisch mit 2.5% B |
| Flussrate: | 1.0 ml/min |
| Detektion: | UV-Detektion bei 230 und 260nm |
| Retentionszeiten: | Butan-2-on: ca. 9.5 min |
| | (1*S*)-Butan-2-ol: ca. 16.6 min |
| | (1*R*)-Butan-2-ol: ca. 18.3 min |

Mit authentischem Material wird eine Eichreihe erstellt, anhand derer die Konzentration unbekannter Proben bestimmt werden kann.

Beispiel 5: Bereitstellung von Glucose-Dehydrogenase zur Cofaktor-Regenerierung und Cofaktorregenerierung mit Glucosedehydrogenase (Enzymkopplung)

Zur Cofaktor-Regenerierung kann Glucose-Dehydrogenase verwendet werden. Das Enzym ist kommerziell erhältlich (z.B. Jülich Fine Chemicals, Bestell Nr. 22.10 oder 19.10). Im folgenden wurde das Glucose-Dehydrogenase-Gen aus *Bacillus subtilis* (Genbank-Acc.No. M12276) verwendet, das in einem E. *coli* XL10 Gold Klon im Plasmid pUC19 kloniert wurde. Dieses Konstrukt trägt die Bezeichnung *E. coli* LU11293.

Zur Fermentation von *E. coli* LU11293 wurde folgendes Medium angesetzt:

| | |
|---|---|
| 560 g | Hefeextrakt (65 %) |
| 448 g | Trypton (Difco) |
| 42 g | $KH_2PO_4$ |
| 84 g | $Na_2HPO_4$ |
| 644 g | Glycerin (99%) |
| 100 mL | SL4 Lösung (5 fach) |
| | |
| 1 g | Tegosipon 3062 |
| | Medium mit Wasser auf 13,5 L auffüllen, pH-Wert |
| | auf 7,0 einstellen, ca. 300 mL für Vorkultur ent- |
| | nehmen, danach 30 min. bei 122°C sterilisieren. |
| | |
| | Sterile Salzlösung* zugeben (vorher die Salzlösung für |
| | die Schüttelkolben entnehmen, siehe Rapport). |
| *Salzlösung:<br>2,1 g $CaCl_2$ * 2 $H_2O$<br>3,5 g $MgSO_4$ * 7 $H_2O$<br>14 g $NH_4Cl$<br>14 mL Ampicillin-Lösung (100 mg/mL)<br>in 500 mL Wasser lösen und sterilfiltrieren | |

Jeweils 150 mL Medium wurden in zwei 1 L Erlenmeyerkolben sterilisiert und mit 5 mL steriler Salzlösung komplettiert. Nach Beimpfen von einer LB-Ampicillin-Agarplatte wurden die Vorkulturen 12 Stunden bei 37°C und 200 UPM inkubiert und zum Fermentationsmedium gegeben. Die Fermentation wurde bei 37˚C, 0,1 bar Innendruck, pH 7,0 (Regelung mit 20% Phosphorsäure und 25% NaOH) mit einer Begasungsrate von 7,5 Umin und 300 upm gestartet (Regelung $pO_2$ zwischen 20 und 50% mit 10-20 L/min Zuluft und 500-1500 Upm). Nach 2 h wurden zur Induktion 0,1 mM IPTG zugegeben und nach insgesamt 13 h die Fermentation beendet. Nach Ernte und Waschen der Zellen (1,3 kg) wurden diese bis zur Verwendung (2-20 g/L im Ansatz) bei -20˚C gelagert.

Equimolare Mengen Glucose und Butan-2-on wurden mit 1-30U/ml Glucosedehydrogenase-Rohextrakt und 1-30U/ml Propanol-Dehydrogenase-Rohextrakt versetzt. 0.02-1 mmol/L NAD bzw. NADP oder NADH bzw. NADPH wurden in Puffer gelöst und bei 10-60˚C inkubiert. Der pH-Wert wurde durch automatische Zugabe von Base konstant gehalten.

Beispiel 6: Cofaktorregenerierung mit Propanol-Dehydrogenase (Substratkopplung)

Die Regenerierung des Cofaktors kann auch durch die Propanol-Dehydrogenase selbst durchgeführt werden. In diesem Fall ist die Zugabe eines gesonderten Regenerationsenzyms nicht notwendig. Die Propanol-Dehydrogenase akzeptiert verschiedene einfache Alkohole als Reduktionsmittel. Sie werden zu den entsprechenden Carbonylverbindungen oxidiert. Ein einfacher Alkohol, der sich zur Regeneration von NADH oder NADPH mit Propanol-Dehydrogenase eignet, ist *iso*-Propanol.

Beispiel 7: Cofaktorregenerierung mit Formiat-Dehydrogenase (Enzymkopplung)

Zur Cofaktor-Regenerierung kann Formiat-Dehydrogenase verwendet werden. Das Enzym ist aus kommerziellen (z.B. Jülich Fine Chemicals Order-No. 09.11, 24.11 oder 25.10) zugänglich. Analog Beispiel 5 kann somit die Regnerierung der Cofaktoren auch mit Formiat-Dehydrogenase erfolgen. Dabei werden equimolare Mengen Formiat und Butan-2-on mit 1-30U/ml Formiat-Dehydrogenase-Rohextrakt und 1-30U/ml Propanol-Dehydrogenase-Rohextrakt eingesetzt. 0.02-1 mmol/L NAD bzw. NADP oder NADH bzw. NADPH wurden in Puffer gelöst und bei 10-60˚C inkubiert. Der pH-Wert wurde durch automatische Zugabe von Säure konstant gehalten.

Beispiel 8: Herstellung von (*S*)-Butan-2-ol mit der rekombinanten Propanol-Dehydrogenase aus *Escherichia coli*

E.coli LU12418 wurden entsprechend Beispiel 2 angezogen, geerntet und aufgeschlossen.

270 g (1.5 mol) D-Glucose, 135 mL (1.5 mol) Butan-2-on, 63 mL GDH-Rohextrakt ($\cong$ 30.5 kU), 95 mL PDH-Rohextrakt ($\cong$ 20 kU) und 400mg (0.6 mmol) NAD oder NADP oder NADH bzw. NADPH wurden in $KP_i$-Puffer (50mM $KP_i$, 1mM $MgCl_2$, pH 6.5) gelöst und bei 30˚C inkubiert. Das Anfangsvolumen des Ansatzes betrug drei Liter. Der pH-Wert wurde durch automatische Zugabe von 2M NaOH konstant gehalten. Abbildung 1 zeigt einen typischen Reaktionsverlauf.

## Patentansprüche

**1.** Verfahren zur Herstellung von (S)-Butan-2-ol durch Reduktion von Butan-2-on in Gegenwart einer Alkohol-Dehydrogenase

> (i) mit der Polypeptidsequenz SEQ ID NO:2, oder
> (ii) mit einer Polypeptidsequenz, die mindestens 80% Sequenzidentität mit SEQ ID NO:2 aufweist.

**2.** Verfahren nach Anspruch 1, **dadurch gekennzeichnet, dass** die Reduktion mit NADH als Cofaktor ausgeführt wird.

**3.** Verfahren nach Anspruch 1, **dadurch gekennzeichnet, dass** der verwendete Cofaktor enzymatisch regeneriert wird.

**4.** Verfahren nach Anspruch 1, **dadurch gekennzeichnet, dass** die Cofaktor-Regenerierung durch Glucose-Dehydrogenase erfolgt.

**5.** Verfahren nach Anspruch 1, **dadurch gekennzeichnet, dass** die Reduktion in wässrigem System durchgeführt wird.

**6.** Verfahren nach Anspruch 1, **dadurch gekennzeichnet, dass** die Alkohol-Dehydrogenase immobilisiert vorliegt.

**7.** Verfahren nach Anspruch 1, **dadurch gekennzeichnet, dass** die verwendete Alkohol-Dehydrogenase in E.coli exprimiert wird.

**8.** Verwendung einer Alkohol-Dehydrogenase

    (iii) mit der Polypeptidsequenz SEQ ID NO:2, oder
    (iv) mit einer Polypeptidsequenz, die mindestens 80 % Sequenzidentität mit SEQ ID NO:2 aufweist,

in einem Verfahren zur Herstellung von (S)-Butan-2-ol durch Reduktion von Butan-2-on.

**Claims**

**1.** A process for preparing (S)-butan-2-ol by reducing butan-2-one in the presence of an alcohol dehydrogenase

    (i) having the polypeptide sequence SEQ ID NO:2, or
    (ii) having a polypeptide sequence which is at least 80% identical to the sequence of SEQ ID NO:2.

**2.** The process according to claim 1, wherein the reduction is carried out using NADH as cofactor.

**3.** The process according to claim 1, wherein the cofactor used is regenerated enzymically.

**4.** The process according to claim 1, wherein the cofactor is regenerated by glucose dehydrogenase.

**5.** The process according to claim 1, wherein the reduction is carried out in an aqueous system.

**6.** The process according to claim 1, wherein the alcohol dehydrogenase is present in an immobilized form.

**7.** The process according to claim 1, wherein the alcohol dehydrogenase used is expressed in E.coli.

**8.** The use of an alcohol dehydrogenase

    (iii) having the Polypeptide sequence SEQ ID NO:2, or
    (iv) having a polypeptide sequence which is at least 80% identical to the sequence of SEQ ID NO:2,

in a process for preparing (S)-butan-2-ol by reducing butan-2-one.

**Revendications**

**1.** Procédé pour à préparation de (S)-butan-2-ol par réduction de butan-2-one en présence d'une alcool-déshydrogénase

    (i) présentant la séquence polypeptididique SEQ ID NO :2, ou
    (ii) présentant une séquence polypeptidique, qui présente une identité de séquence d'au moins 80% avec la SEQ ID NO :2.

**2.** Procédé salon la revendication 1, **caractérisé en ce que** la réduction est réalisée avec du NADPH comme cofacteur.

**3.** Procédé salon la revendication 1, **caractérisé en ce que** le cofacteur utilisé est régénéré enzymatiquement.

**4.** Procédé salon la revendication 1**, caractérisé en ce que** la régénération du cofacteur est réalisée par la glucose-déshydrogénase.

**5.** Procédé selon la revendication 1, **caractérisé en ce que** la réduction est réalisée dans un système aqueux.

**6.** Procédé selon la revendication 1, **caractérisé en ce que** l'alcool-déshydrogénase se trouve sous forme immobilisée.

**7.** Procédé selon à revendication 1, **caractérisé en ce que** l'alcool-déshydrogénase utilisée est exprimée dans E. coli.

**8.** Utilisation d'une alcool-déshydrogénase

(iii) présentant la séquence polypeptididique SEQ ID NO : :2, ou
(iv) présentant une séquence polypeptidique, qui présente une identité de séquence d'au moins 80% avec la SEQ ID NO :2,

dans un procédé pour la préparation de (S)-butan-2-ol par réduction de butan-2-one.

Abbildung 1

Abbildung 1 Reduktion von Butan-2-on zu *S*-Butan-2-ol katalysiert durch die Propanol-Dehydrogenase aus *Escherichia coli*.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1149849 A
- EP 1069183 A
- DE OS10019377 A
- DE 19848129

**In der Beschreibung aufgeführte Nicht-Patentliteratur**

- *J.Org.Chem,* 1992, vol. 57, 1526
- *Chemistry Letters,* 1987, 2089
- *Arch.Biochem Biophys.,* 1992, vol. 292, 539
- *J.Am.Chem.Soc,* 1986, vol. 108, 162
- **NAKAMURA et al.** *Tetrahedron: Asymmetry,* 2003, vol. 14, 2659
- *Tetrahedron: Asymmetry,* 2002, vol. 13, 971
- **VELONIA, K. et al.** Stereospecificity of Hydrogen Transfer by the NAD+-linked Alcohol Dehydrogenase from the Antarctic Psychrophile Moraxella sp. TAE123. *BIOORGANIC & MEDICINAL CHEMISTRY LETTERS,* 1999, vol. 9 (1), 65-68
- *Science,* 1997, vol. 277, 1453
- Reduction of Ketones. **K. NAKAMURA ; T. MATSUDA.** Enzyme Catalysis in Organic Synthesis. Wiley-VCH, 2002, vol. III, 991-1032
- **W. HUMMEL ; K. ABOKITSE ; K. DRAUZ ; C. ROLLMANN ; H. GRÖGER.** *Adv. Synth. Catal.,* 2003, vol. 345 (1, 2), 153-159
- Oxidation of Alcohols. **A. SCHMIDT ; F. HOLLMANN ; B. BÜHLER ; K. DRAUZ ; H. WALDMANN.** Enzyme Catalysis in Organic Synthesis. Wiley-VCH, 2002, vol. III, 991-1032
- Immobilization of Enzymes. **J. LALONDE ; A. MARGOLIN.** Enzyme Catalysis in Organic Synthesis. Wiley-VCH, 2002, vol. III, 991-1032
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. Cold Spring Harbor Laboratory Press, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- Essential Molecular Biology: A Practical Approach. IRL Press at Oxford University Press, 1991
- **AUSUBEL et al.** Current Protocols in Molecular Biology. John Wiley & Sons, 1989
- **GOEDDEL.** Gene Expression Technology: Methods in Enzymology. Academic Press, 1990, vol. 185
- Buch Cloning Vectors. Elsevier, Amsterdam-New York-Oxford, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc. and Wiley Interscience, 1987
- **POUWELS P. H. et al.** Cloning Vectors. Elsevier, 1985
- **F. AUSUBEL et al.** Current Protocols in Molecular Biology. Wiley Interscience, 1997
- **SAMBROOK et al.** Molecular Cloning: A Laboratory Manual. Cold Spring Harbor Laboratory Press, 1989
- **THOMAS, K.R. ; CAPECCHI, M.R.** *Cell,* 1987, vol. 51, 503
- **HARLOW, E. ; LANE, D.** Antibodies: A Laboratory Manual. Cold Spring Harbor (N.Y.) Press, 1988
- Biotechnology. 1993, vol. 3

SEQUENCE LISTING

<110> BASF Aktiengesellschaft

<120> Verfahren zur Herstelllung von (S)-Butan-2-ol

<130> PF 56053

<160> 2

<170> PatentIn version 3.1

<210> 1
<211> 1041
<212> DNA
<213> Escherichia coli

<220>
<221> CDS
<222> (1)..(1041)

<223>

<400> 1

```
atg cag aac atc atc cga aaa gga gga act atg aag gct gca gtt gtt      48
Met Gln Asn Ile Ile Arg Lys Gly Gly Thr Met Lys Ala Ala Val Val
1                   5                   10                  15

acg aag gat cat cat gtt gac gtt acg tat aaa aca ctg cgc tca ctg      96
Thr Lys Asp His His Val Asp Val Thr Tyr Lys Thr Leu Arg Ser Leu
            20                  25                  30

aaa cat ggc gaa gcc ctg ctg aaa atg gag tgt tgt ggt gta tgt cat    144
Lys His Gly Glu Ala Leu Leu Lys Met Glu Cys Cys Gly Val Cys His
        35                  40                  45

acc gat ctt cat gtt aag aat ggc gat ttt ggt gac aaa acc ggc gta    192
Thr Asp Leu His Val Lys Asn Gly Asp Phe Gly Asp Lys Thr Gly Val
        50                  55                  60
```

```
att ctg ggc cat gaa ggc atc ggt gtg gtg gca gaa gtg ggt cca ggt        240
Ile Leu Gly His Glu Gly Ile Gly Val Val Ala Glu Val Gly Pro Gly
65              70                  75                  80

gtc acc tca tta aaa cca ggc gat cgt gcc agc gtg gcg tgg ttc tac        288
Val Thr Ser Leu Lys Pro Gly Asp Arg Ala Ser Val Ala Trp Phe Tyr
                85                  90                  95

gaa gga tgc ggt cat tgc gaa tac tgt aac agt ggt aac gaa acg ctc        336
Glu Gly Cys Gly His Cys Glu Tyr Cys Asn Ser Gly Asn Glu Thr Leu
            100                 105                 110

tgc cgt tca gtt aaa aat gcc gga tac agc gtt gat ggc ggg atg gcg        384
Cys Arg Ser Val Lys Asn Ala Gly Tyr Ser Val Asp Gly Gly Met Ala
            115                 120                 125

gaa gag tgc atc gtg gtc gcc gat tac gcg gta aaa gtg cca gat ggt        432
Glu Glu Cys Ile Val Val Ala Asp Tyr Ala Val Lys Val Pro Asp Gly
        130                 135                 140

ctg gac tcg gcg gcg gcc agc agc att acc tgt gcg gga gtc acc acc        480
Leu Asp Ser Ala Ala Ala Ser Ser Ile Thr Cys Ala Gly Val Thr Thr
145                 150                 155                 160

tac aaa gcc gtt aag ctg tca aaa att cgt cca ggg cag tgg att gct        528
Tyr Lys Ala Val Lys Leu Ser Lys Ile Arg Pro Gly Gln Trp Ile Ala
                165                 170                 175

atc tac ggt ctt ggc ggt ctg ggt aac ctc gcc ctg caa tac gcg aag        576
Ile Tyr Gly Leu Gly Gly Leu Gly Asn Leu Ala Leu Gln Tyr Ala Lys
            180                 185                 190

aat gtc ttt aac gcc aaa gtg atc gcc att gat gtc aat gat gag cag        624
Asn Val Phe Asn Ala Lys Val Ile Ala Ile Asp Val Asn Asp Glu Gln
        195                 200                 205

tta aaa ctg gca acc gaa atg ggc gca gat tta gcg att aac tca cac        672
Leu Lys Leu Ala Thr Glu Met Gly Ala Asp Leu Ala Ile Asn Ser His
        210                 215                 220

acc gaa gac gcc gcc aaa att gtg cag gag aaa act ggt ggc gct cac        720
Thr Glu Asp Ala Ala Lys Ile Val Gln Glu Lys Thr Gly Gly Ala His
225                 230                 235                 240

gct gcg gtg gta aca gcg gta gct aaa gct gcg ttt aac tcg gca gtt        768
Ala Ala Val Val Thr Ala Val Ala Lys Ala Ala Phe Asn Ser Ala Val
                245                 250                 255

gat gct gtc cgt gca ggc ggt cgt gtt gtg gct gtc ggt cta ccg ccg        816
Asp Ala Val Arg Ala Gly Gly Arg Val Val Ala Val Gly Leu Pro Pro
            260                 265                 270

gag tct atg agc ctg gat atc cca cgt ctt gtg ctg gat ggt att gaa        864
Glu Ser Met Ser Leu Asp Ile Pro Arg Leu Val Leu Asp Gly Ile Glu
        275                 280                 285

gtg gtc ggt tcg ctg gtc ggc acg cgc cag gat tta act gaa gcc ttc        912
Val Val Gly Ser Leu Val Gly Thr Arg Gln Asp Leu Thr Glu Ala Phe
        290                 295                 300

cag ttt gcc gcc gaa ggt aaa gtg gtg ccg aaa gtc gcc ctg cgt ccg        960
```

17

```
Gln Phe Ala Ala Glu Gly Lys Val Val Pro Lys Val Ala Leu Arg Pro
305             310             315             320

tta gcg gac atc aac acc atc ttt act gag atg gaa gaa ggc aaa atc    1008
Leu Ala Asp Ile Asn Thr Ile Phe Thr Glu Met Glu Glu Gly Lys Ile
                325             330             335

cgt ggc cgc atg gtg att gat ttc cgt cac taa                        1041
Arg Gly Arg Met Val Ile Asp Phe Arg His
            340             345
```

<210> 2
<211> 346
<212> PRT
<213> Escherichia coli

<400> 2

```
Met Gln Asn Ile Ile Arg Lys Gly Gly Thr Met Lys Ala Ala Val Val
1               5               10              15

Thr Lys Asp His His Val Asp Val Thr Tyr Lys Thr Leu Arg Ser Leu
            20              25              30

Lys His Gly Glu Ala Leu Leu Lys Met Glu Cys Cys Gly Val Cys His
            35              40              45

Thr Asp Leu His Val Lys Asn Gly Asp Phe Gly Asp Lys Thr Gly Val
        50              55              60

Ile Leu Gly His Glu Gly Ile Gly Val Val Ala Glu Val Gly Pro Gly
65              70              75              80

Val Thr Ser Leu Lys Pro Gly Asp Arg Ala Ser Val Ala Trp Phe Tyr
                85              90              95

Glu Gly Cys Gly His Cys Glu Tyr Cys Asn Ser Gly Asn Glu Thr Leu
            100             105             110

Cys Arg Ser Val Lys Asn Ala Gly Tyr Ser Val Asp Gly Gly Met Ala
            115             120             125

Glu Glu Cys Ile Val Val Ala Asp Tyr Ala Val Lys Val Pro Asp Gly
        130             135             140

Leu Asp Ser Ala Ala Ala Ser Ser Ile Thr Cys Ala Gly Val Thr Thr
```

```
        145                    150                    155                    160

        Tyr Lys Ala Val Lys Leu Ser Lys Ile Arg Pro Gly Gln Trp Ile Ala
                        165                    170                    175

        Ile Tyr Gly Leu Gly Gly Leu Gly Asn Leu Ala Leu Gln Tyr Ala Lys
                        180                    185                    190

        Asn Val Phe Asn Ala Lys Val Ile Ala Ile Asp Val Asn Asp Glu Gln
                        195                    200                    205

        Leu Lys Leu Ala Thr Glu Met Gly Ala Asp Leu Ala Ile Asn Ser His
                210                    215                    220

        Thr Glu Asp Ala Ala Lys Ile Val Gln Glu Lys Thr Gly Gly Ala His
        225                    230                    235                    240

        Ala Ala Val Val Thr Ala Val Ala Lys Ala Ala Phe Asn Ser Ala Val
                        245                    250                    255

        Asp Ala Val Arg Ala Gly Gly Arg Val Val Ala Val Gly Leu Pro Pro
                        260                    265                    270

        Glu Ser Met Ser Leu Asp Ile Pro Arg Leu Val Leu Asp Gly Ile Glu
                        275                    280                    285

        Val Val Gly Ser Leu Val Gly Thr Arg Gln Asp Leu Thr Glu Ala Phe
                290                    295                    300

        Gln Phe Ala Ala Glu Gly Lys Val Val Pro Lys Val Ala Leu Arg Pro
        305                    310                    315                    320

        Leu Ala Asp Ile Asn Thr Ile Phe Thr Glu Met Glu Glu Gly Lys Ile
                        325                    330                    335

        Arg Gly Arg Met Val Ile Asp Phe Arg His
                        340                    345
```